# EUROPEAN PATENT APPLICATION

(11) **EP 2 465 848 A1**
(43) Date of publication of application: **20.06.2012**
(21) Application number: 10194913.9
(22) Date of filing: 14.12.2010
(51) Int. Cl.: C07D 211/42, C07D 403/04, C07F 7/10, C07F 7/18

(54) **Triazolylpiperidine derivatives and method for making the same**

(71) Applicant: Bergen Teknologioverføring AS, 5006 Bergen (NO)
(72) Inventor: Sydnes, Leiv K., 5239 Radal (NO); Haug, Bengt Erik, 5114 Tertnes (NO); Harmsen, Rianne, 5032 Bergen (NO)
(74) Representative: Kröncke, Rolf

(57) **Abstract**

In a first aspect, the present invention relates to a method for the preparation of alkynylpiperidine and alkynylpiperidine derivatives. In particular, a method is provided for preparing alkynylpiperidine derivatives starting from epoxide compounds. Further, the alkynylpiperidine derivatives according to the present inventions are substituted trans-3- 4-alkynylpiperidine derivatives. In another aspect, triazolylpiperidine compounds and methods for said preparation are provided. In particular, the triazolylpiperidine compounds according to the present invention are obtained by reacting an alkynylpiperidine derivatives with an organic azid, preferably, in the presence of a Cu (I) or Ru catalyst. In a preferred embodiment, 1,4-disubstituted or 1,5-disubstituted 1,2,3-triazolylpiperidine compounds are provided.

## Description

In a first aspect, the present invention relates to a method for the preparation of alkynylpiperidine and alkynylpiperidine derivatives. In particular, a method is provided for preparing alkynylpiperidine derivatives starting from epoxide compounds. Further, the alkynylpiperidine derivatives according to the present inventions are substituted trans-3,4-alkynylpiperidine derivatives. In another aspect, triazolylpiperidine compounds and methods for said preparation are provided. In particular, the triazolylpiperidine compounds according to the present invention are obtained by reacting an alkynylpiperidine derivative with an organic azide, preferably, in the presence of a Cu (I) or Ru catalyst. In a preferred embodiment, 1,4-disubstituted or 1,5-disubstituted 1,2,3-triazolylpiperidine compounds are provided.

### Prior art

Piperidine moieties are considered as one of the privileged structures in medicinal chemistry. These are small molecules capable of providing high affinity ligands for multiple receptors and enzymes (Mason, J.S. et.al., J Med Chem 1999, 42, (17), 3251-3264; Costantino, L. et.al., Curr Med Chem 2006, 13, (1), 65-85) and of these structures the piperidine ring is among the most common heterocycles occurring in pharmaceuticals. The vast number of substituted piperidine derivatives among biologically active compounds has motivated the development of a multitude of synthetic routes towards these structures, and these endeavours have yielded new routes to already known piperidines and also provided medicinal chemists with new building blocks e.g. Schramm, H. et.al., Synthesis 2009, (10), 1659-1662; Schramm, H. et.al., Eur J Org Chem 2010, 2010, (9), 1745-1753. In recent years, numerous reports on the facile and regioselective synthesis of disubstituted 1,2,3-triazoles from terminal alkynes and organic azides have appeared in the literature.

There is still an ongoing need for new piperidine derivatives, thus, providing useful scaffolds for pharmaceutically active compounds, e.g. having high affinity to receptors and enzymes as described for known piperidine compounds in the art.

Hence, one object of the present invention is to provide new methods and compounds based on a piperidine scaffold, in particular, providing alkynylpiperidine derivatives useful as intermediate compounds and starting material for the preparation of optionally substituted triazolylpiperidine derivatives. In another aspect, the object of the present invention is to provide new optionally substituted triazolylpiperidine compounds useful as scaffolds for new active pharmaceutical ingredients.

### Summary of the present invention

In a first aspect, the present invention relates to a compound of the general formula I 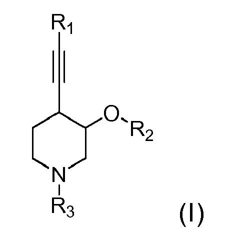 wherein R₁ is hydrogen or a protecting group,

R₂ and R₃ are independently from each other hydrogen or a protection group, or an C₁-C₁₈ alkyl group, a C₂-C₁₈ alkenyl group, a C₃-C₁₈ aryl group, a C₃-C₁₈ heteroalkyl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents, preferably,R₁ and R₂ are not hydrogen at the same time, with the proviso that R₁ and R₂ are not hydrogen at the same time when the substituents at position 3 and position 4 of the piperidine ring are in cis-form

In a preferred embodiment, the alkynylpiperidine derivative of general formula I is an ethynylpiperidine derivative wherein substituent R1 is hydrogen.

In another aspect, the present invention provides a method for the synthesis of a compound according to general formula I including the step of reacting an epoxide according to general formula II 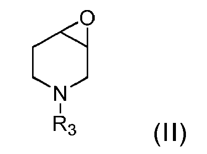 wherein R₃ is a protection group
with a protected acetylide derivative, which is prepared by deprotonating a protected acetylene derivative using an alkyllithium compound, a Grignard reagent, a lithium dialkylamide or hexamethyldisilazide, preferably n-BuLi, in the presence of a trialkylaluminium reagent, like AlMe₃, and
b) cooling the reaction mixture at a temperature below room temperature, preferably at -78 °C;
c) adding a Lewis acid, preferably, BF₃ x OEt₂, and
d) reacting the same for obtaining a compound of general formula I
wherein R₂ is hydrogen.

In another aspect, an optionally substituted triazolylpiperidine compound of general formulas III or IV are provided. The compound of general formula III is a 1,4-disubstituted 1,2,3- triazolylpiperidine compound whereas general formula IV represents a substituted 1,5-disubstituted 1,2,3-triazolylpiperidine derivative.

In another aspect, the present invention provides a method for producing an optionally substituted triazolylpiperidine according to general formula III or IV according to the present invention. Said method comprising the step of reacting a compound of general formula I with an organic azide derivative to obtain a compound of general formula III or IV.

### Brief description of the drawings

Figure 1 shows the preparation of alkynylpiperidine and derivates thereof according to general formula I.

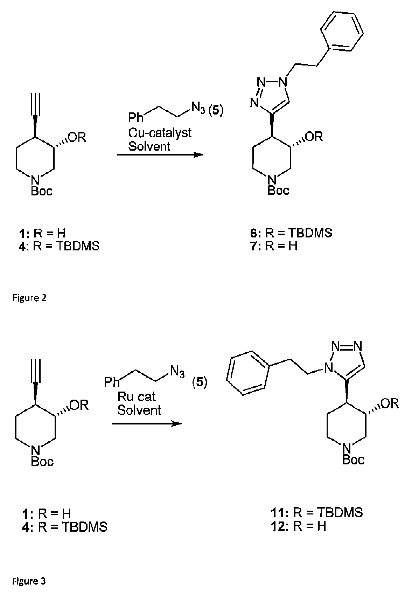

In figure 2 the Cu(I) catalyzed 1,3-dipolar cycloaddition of an organic azide to a compound of general formula I is shown.
In figure 3 the Ru catalyzed 1,3-dipolar cycloaddition of an organic azide to a compound of general formula I is shown obtaining a 1,5-disubstituted 1,2,3-triazolylpiperidine derivative.
In figure 4a and 4b (general formula III) and 4c (general formula IV) preferred embodiments of the triazolylpiperidine derivatives are shown.

### Detailed description of the present invention

In a first aspect, the present invention relates to a compound of general formula I 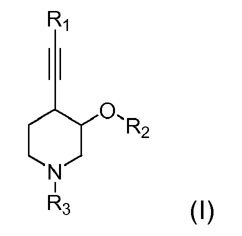 wherein R₁ is hydrogen or a protecting group, R₂ and R₃ are independently from each other hydrogen or a protection group, or an C₁-C₁₈ alkyl group, a C₂-C₁₈ alkenyl group, a C₃-C₁₈ aryl group, a C₃-C₁₈ heteroalkyl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkenyl, hydroxyl,carbamoyl, C₁ to C₈ alkylcarbamoyl, amido, alkoxy, alkoxycarbonyl, alkylcarbamoyloxy, alkoxycarbonylamino, amino, alkanoyloxy group, alkylthio and sulphur-containing analogs of oxygen containing substituents, preferably, R₁ and R₂ are not hydrogen at the same time, with the proviso that R₁ and R₂ are not hydrogen at the same time when the substituents at position 3 and position 4 of the piperidine ring are in cis-form.

The term "C1 to C18" as used herein include compounds having C1, C2, C3, C4, C5, C6, C7, C8, C9, C10, C11, C12, C13, C14, C15, C16, C17, or C18 carbon atoms. The term "C1 to C6" include C1, C2, C3, C4, C5, C6 carbon atoms. The term "C2 to C6" include, C2, C3, C4, C5, C6 carbon atoms. The groups may be present in linear, branched or cyclic form.

"Alkyl" refers to a saturated aliphatic hydrocarbon including straight chain and branched chain groups. "Alkyl" may be exemplified by groups such as methyl, ethyl, n-propyl, isopropyl, n-butyl and the like. Alkyl groups may be substituted or unsubstituted. Substituents may also be themselves substituted. When substituted, the substituent group is preferably, but not limited to, C₁-C₄ alkyl, aryl, amino, cyano, halogen, alkoxy or hydroxyl.

"Alkenyl" refers to an unsaturated aliphatic hydrocarbon moiety including straight chain and branched chain groups. Alkenyl moieties contain at least one alkene. "Alkenyl" may be exemplified by groups such as ethenyl, n-propenyl, isopropenyl, n-butenyl and the like. Alkenyl groups may be substituted or unsubstituted. Substituents may also be themselves substituted. When substituted, the substituent group is preferably, but not limited to alkyl, halogen or alkoxy. Substituents may also be themselves substituted. Substituents may be located on the alkene itself and also on the adjacent member atoms of the alkenyl moiety.

"Alkynyl" refers to an unsaturated aliphatic hydrocarbon moiety including straight chain and branched chain groups. Alkynyl moieties contain at least one alkyne. "Alkynyl" may be exemplified by groups such as ethynyl, propynyl, n-butynyl and the like. Alkynyl groups may be substituted or unsubstituted. When substituted, the substituent group is preferably alkyl, amino, cyano, halogen, alkoxyl or hydroxyl. Substituents may also be themselves substituted. Substituents are not on the alkyne itself but on the adjacent member atoms of the alkynyl moiety.

"Acyl" or "carbonyl" refers to the group -C(O)R wherein R is H, alkyl, alkenyl, alkynyl, aryl, heteroaryl, carbocyclic, heterocarbocyclic, C₁-C₄ alkyl aryl or C₁-C₄ alkyl heteroaryl.

"Alkoxy" refers to the group -O-R wherein R is acyl, alkyl alkenyl, alkyl alkynyl, aryl, carbocyclic, heterocarbocyclic, heteroaryl, C₁-C₄ alkyl aryl or C₁-C₄ alkyl heteroaryl.

"Aralkyl" refers to a radical in which an aryl group is substituted for a hydrogen atom of an alkyl group; e.g., C₆H₅CH₂-.

"Amino" refers to the group -NR'R" wherein R' and R" are each, independently, hydrogen, alkyl, aryl, heteroaryl, C₁-C₄ alkyl aryl or C₁-C₄ alkyl heteroaryl. The R' and R" groups may themselves be linked to form a ring.

"Aryl" refers to an aromatic carbocyclic group. "Aryl" may be exemplified by phenyl. The aryl group may be substituted or unsubstituted. Substituents may also be themselves substituted. When substituted, the substituent group is preferably, but not limited to, alkyl, alkoxy, heteroaryl, acyl, carboxyl, carbonylamino, nitro, amino, cyano, halogen or hydroxyl. The substituents may be positioned at various locations on an aryl group. For example, substituents on a phenyl group may be located at an ortho-position, a meta-position, the paraposition, or combinations thereof.

"Carboxyl" refers to the group -C(=O)OR, where R is a C₁-C₄ alkyl, aryl, The alkyl or aryl group may be substituted or unsubstituted. Substituents may also be themselves substituted. When substituted, the substituent group is preferably, but not limited to, alkyl, alkoxy, heteroaryl, acyl, carboxyl, carbonylamino, nitro, amino, cyano, halogen or hydroxyl. The substituents may be positioned at various locations on an aryl group.

"Carbonyl" refers to the group -C(O)R wherein each R is, independently, hydrogen, alkyl, aryl, cycloalkyl, heterocycloalkyl, heteroaryl, C₁-C₄ alkyl aryl or C₁-C₄ alkyl heteroaryl.

"Heteroaryl" or "heteroaromatic" refers to a monocyclic or bicyclic aromatic carbocyclic radical having one or more heteroatoms in the carbocyclic ring. Heteroaryl may be substituted or unsubstituted. When substituted, the substituents may themselves be substituted. Preferred, but non-limiting substituents, are aryl, C1-C4 alkyl aryl, amino, halogen, hydroxyl, cyano, nitro, carboxyl, carbonylamino or C1-C4 alkyl. Preferred heteroaromatic groups include tetrazoyl, thiazolyl, thienyl, thiophenyl, thiazolyl, purinyl, pyrimidyl. pyridyl, and furanyl. More preferred heteroaromatic groups include benzothiofuranyl, thiophenyl, thienyl, furanyl, tetrazoyl, triazolyl, and pyridyl.

"Heteroatom" means an atom other than carbon in the ring of a heterocyclic group or a heteroaromatic group or the chain of a heterogeneous group. Preferably, heteroatoms are selected from the group consisting of nitrogen, sulfur, phosphor and oxygen atoms. Groups containing more than one heteroatom may contain different heteroatoms.

"Heterocarbocyclic group" or "heterocycloalkyl" or "heterocyclic" means a monovalent saturated or unsaturated hydrocarbon ring containing at least one heteroatom. Heterocarbocyclic groups are monocyclic, or are fused, spiro, or bridged bicyclic ring systems. Monocyclic heterocarbocyclic groups contain 3 to 10 carbon atoms, preferably 4 to 7 carbon atoms, and more preferably 5 to 6 carbon atoms in the ring. Bicyclic heterocarbocyclic groups contain 8 to 12 carbon atoms, preferably 9 to 10 carbon atoms in the ring. Heterocarocyclic groups may be substituted or unsubstituted. Suitable substituents include, but are not limited to, lower alkyl, hydroxyl, nitrile, halogen and amino. Substituents may also be themselves substituted. Preferred heterocarbocyclic groups include epoxy, tetrahydrofuranyl, azacyclopentyl, azacyclohexyl, piperidyl, and homopiperidyl. More preferred heterocarbocyclic groups include piperidyl, and homopiperidyl. The most preferred heterocarbocyclic group is piperidyl. Heterocarbocyclic groups are not aromatic.

"Thioalkyl" refers to the group -S-alkyl.

That is, the present inventors were successful in obtaining compounds of the general formula I which represents useful as starting material and intermediates in the preparation of substituted piperidine derivatives, like triazolyl-substituted piperidine derivatives. In a preferred embodiment, the substituent R₁ is hydrogen and R₂ and R₃ are different protection groups. For example, R₁ is hydrogen and R₂ is a silyl protecting group while R₃ is a carbamate protecting group, preferably a tert-butoxycarbonyl group, or a sulfonamide group, or any other suitable amine protecting group described in the art.

The term "protecting group" refers to a group used to protect a certain functional group in a chemical synthesis as described in the art (P.J. Kocienski, Protecting groups, ISBN 9783131356031, Thieme publishing group), preferred protecting groups according to the present invention include tert-butoxycarbonyl, benzyloxycarbonyl, tosyl, nosyl, trimethylsilyl, dimethyl-tert-butylsilyl, When more than one protecting groups are present in the compounds, it is preferred that the protecting groups are different from each other.

The compounds of the general formula I are useful as intermediates in the production of triazolyl-substituted piperidine compounds.

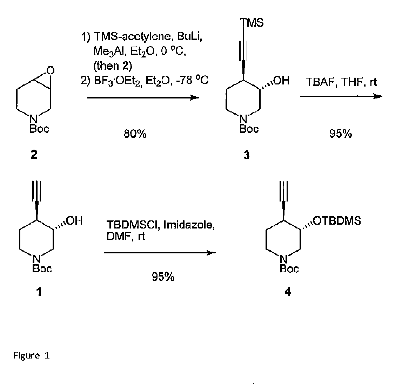

The compound of the general formula I may be prepared as shown in figure 1.

That is, as a starting material an epoxide derivative of a piperidine compound as shown in general formula II 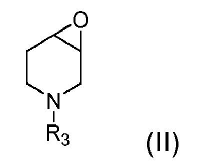 is used as a starting material. Therein, R₃ represents a protection group. The epoxide is reacted with a protected acetylide derivative, which is prepared by deprotonating a protected acetylene derivative using an alkyllithium compound, a Grignard Reagent, a lithium dialkylamide or hexamethyldisilazide, preferably n-BuLi, in the presence of a trialkylaluminium reagent, preferably AIMe₃.Thereafter, the reaction mixture is cooled at a temperature below room temperature, preferably at -78 °C.

With the term "protected acetylene derivative" is meant an acetylene derivative where one of the hydrogen atoms of acetylene is substituted with a protecting group like trimethylsilyl.

In a next step the epoxide is activated with a Lewis acid, preferably, BF₃ x OEt₂, thus, allowing a ring opening in order to obtain a compound of general formula I wherein R₂ is hydrogen.

That is, nucleophilic ring opening of an epoxide of general formula II can afford two different regioisomers, depending on the reagents and conditions used. Under neutral or basic conditions, formation of a 1:1 mixture of isomers is expected. However, if the reaction is carried out under acidic conditions, the formation of a cationic intermediate has been invoked to enhance the directing effect of the nitrogen atom of the piperidine ring. As a consequence the nucleophile will attack position 4 of the piperidine ring and afford only one regioisomer. By using a Lewis acid to coordinate the epoxide oxygen, thereby creating a cationic intermediate, the ethynyl nucleophile could be directed towards the 4-position of the piperidine. Thus, it is possible to synthesize specifically trans 3,4-disubstituted piperidine derivatives. In a preferred embodiment of the present invention, the reaction is carried out under conditions using a Lewis acid, thus, resulting in trans-3.4-disubstituted piperidine derivatives.

The ring opening of epoxide of general formula II (figure 1) with lithium TMS-acetylide was carried out under a set of conditions, but in all cases using n-BuLi to deprotonate the TMS-acetylene as shown in the examples. By adding AlMe₃ to the lithium TMS acetylide (presumably forming the aluminate) and further activating of the epoxide with BF₃ x OEt₂, a clean ring opening took place and furnished alcohol 3 in high yield (figure 1). However, ring opening using TMS-acetylene, n-BuLi and Me₃Al only gave no reaction, just recovered starting material; thus, the Lewis acid, like BF₃ x OEt₂ plays a crucial role in the reaction. Finally, the TMS was removed using e.g. by TBAF, to give tert-butoxycarbonyl-protected 4-ethynyl-3-hydroxy piperidine (1) in excellent yield.

In all cases where ring opening of the epoxide 2 occurred, only the product resulting from nucleophilic attack at the piperidine 4-position could be isolated. The regioselectivity was confirmed by X-ray single structure of 3, which also revealed that the piperidine substituents at C-3 and C-4 were situated trans to each other. Further details are given in the experimental section. Both the substituent at C-3 and C-4 are oriented in axial positions. The N-Boc protecting group is oriented in a pseudoequatorial position. In the X-ray structure, two enantiomers of the piperidine derivative are hydrogen bonded through the hydroxyl groups and the carbonyl oxygen atoms of the N-Boc protecting group (data not shown).

Hence, in a preferred embodiment, the compound of general formula I is a compound wherein the substituents at positions 3 and 4 of the piperidine ring, namely the residue OR₂ and ethynyl-R, are in trans position.

In a preferred embodiment, the method for preparing a compound according to general formula I according to any one of claims 1 to 3 comprising the step of
a) reacting an epoxide according to general formula II 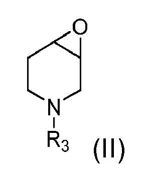 wherein R₃ is a protection group with a protected acetylide derivative, which is prepared by deprotonating a protected acetylene derivative using an alkyllithium compound, a Grignard reagent, a lithium dialkylamide or hexamethyldisilazide, preferably n-BuLi, in the presence of a trialkylaluminium reagent, like AlMe₃, and
b) cooling the reaction mixture at a temperature below room temperature, preferably at -78 °C;
c) adding a Lewis acid, preferably, BF₃ x OEt₂, and
d) reacting the same for obtaining a compound of general formula I wherein R₂ is hydrogen.

Preferably, the method further comprises the step of reacting the compound obtained in step d) with a protecting agent to protect the compound of general formula I at substituent R₂ for obtaining a compound of general formula I with R₁ being hydrogen and R₂ is a protection group.

In a preferred embodiment, the reaction with the protected acetylene derivative is effected at a temperature of from 0 °C to room temperature and/or the reaction with the Lewis acid, in particular, BF₃ x OEt₂, is conducted at a temperature below -50 °C, preferably below -70 °C, for example, at -78 °C.

In another preferred embodiment, the method results in a compound of general formula I wherein the substituents at position 3 and 4 of the piperidine ring are in trans position.

In another aspect, the present invention relates to an optionally substituted triazolylpiperidine compound of general formula III or IV 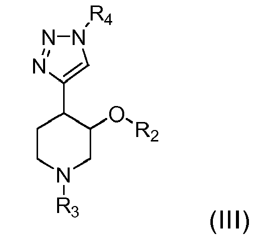 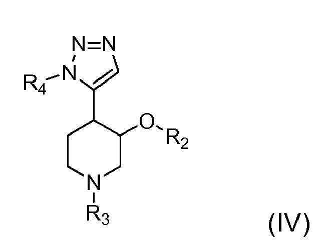 wherein R₄ is an C₁-C₁₈ alkyl group, a C₂-C₁₈ alkenyl group, a C₃-C₁₈ aryl group, a C₃-C₁₈ heteroalkyl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkenyl, hydroxyl,carbamoyl, C₁ to C₈ alkylcarbamoyl, amido, alkoxy, alkoxycarbonyl, alkylcarbamoyloxy, alkoxycarbonylamino, amino, alkanoyloxy group, alkylthio and sulphur-containing analogs of oxygen containing substituents, R₂ and R₃ are defined as above.

The compound of general formula III represents a 1,4-disubstituted 1,2,3-triazolylpiperidine derivative while the compound of general formula IV represents a 1,5-disubstituted 1,2,3-triazolylpiperidine derivative.

In a preferred embodiment of the optionally substituted triazolylpiperidine compound according to the present invention, the substituent R₂ is selected from the group of hydrogen, C₁-C₈ alkyl, a benzyl group, a naphthyl group, a carbonylbenzyl group, or a carbonylnaphthyl group. In another preferred embodiment, substituent R₃ is hydrogen.

Furthermore, it is preferred that substituent R₄ is selected from an aryl group optionally being substituted with an alkoxy group like a benzyl group. In particular preferred, the substituent R₄ is an arylethyl group, in particular, a phenethyl group optionally substituted with a methoxy group. Alternatively, R₄ is a phenyl group optionally substituted with a methoxy group.

In another preferred embodiment, the compound of general formula III or IV is a compound wherein the substituent R₂ is a benzyl group or a naphthenmethyl group.

Moreover, another preferred embodiment of the compounds of general formula III or IV relates to compounds in the trans form at position 3 and 4 of the piperidine moiety.

Moreover, the present invention relates to a method for obtaining an optionally substituted triazolylpiperidine compound of general formula III or IV. Said method comprises the step of reacting a compound of general formula I wherein R₁ and R₂ are as defined herein or, alternatively, both R₁ and R₂ are hydrogen, with an organic azide, thus, obtaining a compound of general formula III or IV. It is preferred that the compound of general formula I is a compound wherein R₁ is hydrogen while R₂ is hydrogen or a protecting group, like TBDMS (tert-butyldimethylsilyloxy).

It is preferred that the reaction for obtaining the optionally substituted triazolylpiperidine according to the present invention is conducted in the presence of a catalyst, in particular of a Cu(I) catalyst or a Ru catalyst.

In particular, it is possible to obtain a 1,4-disubstituted 1,2,3-triazolylpiperidine of general formula III when reacting the compound of general formula I with an organic azide in the presence of a Cu(I) catalyst. Further, it is possible to obtain a 1,5-disubstituted 1,2,3-triazolylpiperidine when conducting the reaction in the presence of a Ru catalyst.

The reactions of acetylenes 1 and 4 with organic azides were conducted with the aim of getting triazoles. This reactions belong to the Huisgen 1,3-dipolar cycloadditions which generally give a mixture of the 1,4- and the 1,5-disubstituted 1,2,3-triazole (Huisgen, R., In 1,3-Dipolar cycloaddition chemistry, ed.; Padwa, A., Wiley: New York 1984, 1-176).

Based on recent advances in this field has lead to the discovery of metalcatalyzed reactions in which both 1,4- and 1,5-disbustituted 1,2,3-triazoles can be formed selectively. Cu(I) catalysis regiospecifically combines terminal acetylenes and organic azides to give only 1,4-disubstituted 1,2,3-triazoles (Tornøe, C.W. et.al., J Org Chem 2002, 67, (9), 3057-3064), whereas Ru catalysis preferably yields 1,5-disbustituted 1,2,3-triazoles (Zhang, L. et.al., J Am Chem Soc 2005, 127, (46), 15998-15999).

In the following, the invention will be described further in the examples. The examples are not intended to restrict the claimed matter but are used only to illustrate the invention further.

### Examples

### Example 1

### Synthesis of compounds of general formula (II) in the presence of Cu (I) catalyst

Firstly, the t-BuOH-water protocol developed by Sharpless and coworkers (Rostovtsev, V.V. et.al., Angew Chem Int Edit 2002, 41, (14), 2596-2599) was tried but this turned out to give 1,4-disbustituted 1,2,3-triazole 6 from the TBDMS protected 4 in 32% isolated yield only after 24 hrs reaction time (Table 1, entry 1); in addition 55% of the starting material was recovered. Changing the solvent to a CH₂Cl₂-water mixture dramatically increased the yield of 6 (Table 1, entry 2). When the same reaction was carried out with the unprotected alcohol 1, the 1,4-disbustituted 1,2,3-triazole 7 was isolated in high yield (Table 1, entry 3). Use of CHCl₃-water as the solvent gave a comparable result (Table 1, entry 4). By using a Cu(I) salt, instead of in situ generating Cu(I) from Cu(II)SO₄ and sodium ascorbate, and acetonitrile as the solvent at elevated temperature, triazole 7 could be isolated in 90% yield (Table 1, entry 5). Since acetonitrile was used as solvent, no base was added to stabilize the Cu(I) as has been described in the literature.

**Table 1: 1,4-Disubstituted 1,2,3-triazoles prepared by reacting acetylene 3 or 4 with 2-(azidoethyl)benzene (5)**

| Entry | R | Solvent | Catalyst | Reaction temperature | Product | Isolated yield (%) |
|---|---|---|---|---|---|---|
| 1 | TBDMS | t-BuOH / H₂O | CuSO₄ / NaAsc | r.t. | 6 | 32 |
| 2 | TBDMS | CH₂Cl₂ / H₂O | CuSO₄ / NaAsc | r.t. | 6 | 84 |
| 3 | H | CH₂Cl₂ / H₂O | CuSO₄ / NaAsc | r.t. | 7 | 78 |
| 4 | H | CHCl₃ / H₂0 | CuSO₄ / NaAsc | r.t. | 7 | 75 |
| 5 | H | MeCN | Cul | 60 °C | 7 | 90 |

Because of their often small size, organic azides can be unstable and difficult to handle, thus in situ generation of organic azides from the corresponding halides by nucleophilic displacement with sodium azide can be advantageous (scheme 1). This principle can be applied for three-component syntheses of 1,2,3-triazoles.

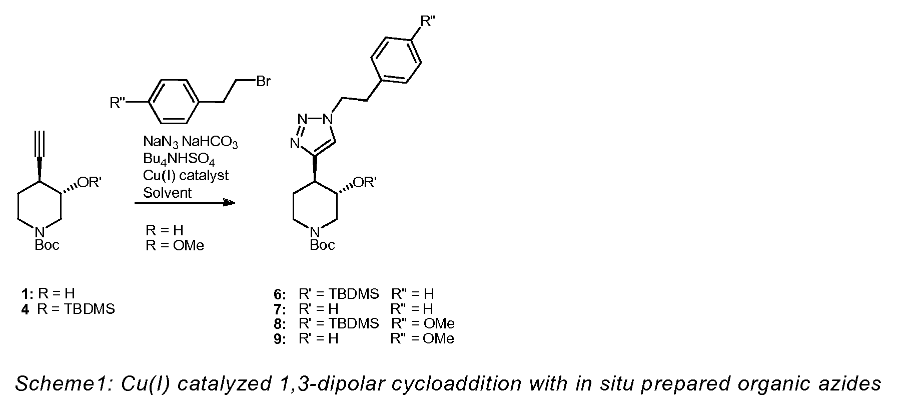

Using acetonitrile-water as solvent for the in situ generation of the azide with Cul as the catalyst for the 1,3-dipolar cycloaddition (Tabel 2, entry 7) gave as major product the diyne of compound 1 and triazole 7 as minor product (scheme 4). The diyene is presumably formed by terminal alkyne homocoupling (the Glaser alkyne homocoupling). Homocoupling was not observed when 7 was prepared from 1 and 2-phenylethyl azide using Cul in acetonitrile (Table 1, entry 5). This homocoupling is an oxidative dimerization, therefore high amount of oxygen are needed. When the azide is generated in situ, water is used as co-solvent which results in formation of the diyene 10.

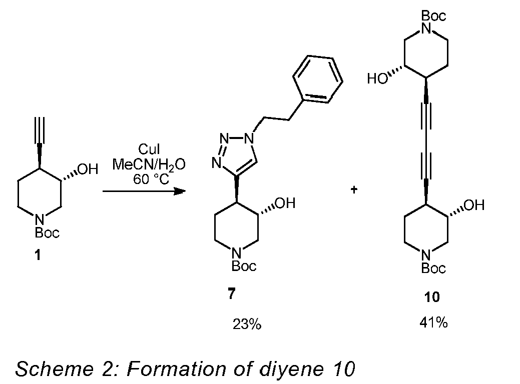

First the azide was prepared from the bromide using NaN₃ and Bu₄NHSO₄ as phase transfer catalyst. Next the piperidine moiety and the catalyst were added to the solution to obtain the 1,4-disubstituted 1,2,3-triazole compound (scheme 3). For the preparation of compound 6 (Table 2, entry 1 and 2) incomplete conversion was observed, even after 24 hrs, and starting material was recovered. Using the t-BuOH/H₂O as solvent (Table 2, entry 3) did not give any reaction even at 60 °C and the starting material was recovered. For the preparation of triazole 8 (entry 4 and 5) increasing the temperature gave a significant increase in yield over the same reaction time (24 hrs). a similar result was obtained in the synthesis of triazole 9 using the same conditions (Table 2, entry 7).

**Table 2: 1,4-Disubstituted 1,2,3-triazoles prepared by reacting acetylene 3 or 4 with in situ prepared azides**

| Entry | R' | R" | Solvent | Catalyst | Reaction temperature | Product | Isolated yield (%) |
|---|---|---|---|---|---|---|---|
| 1 | TBDMS | H | CHCl₃ / H₂O (1:2) | CuSO₄ / NaAsc | r.t. | 6 | 30 |
| 2 | TBDMS | H | MeCN | CuSO₄ / NaAsc | r.t. | 6 | 16 |
| 3 | TBDMS | H | t-BuOH / H₂O (1:1) | CuSO₄ / NaAsc | 60 °C | No reaction | |
| 4 | TBDMS | OMe | CHCl₃ / H₂O (1:2) | CuSO₄ / NaAsc | r.t. | 8 | 9 |
| 5 | TBDMS | OMe | CHCl₃ / H₂O (1:2) | CuSO₄ / NaAsc | 80 °C | 8 | 63 |
| 6 | H | OMe | CHCl₃ / H₂O (1:2) | CuSO₄ / NaAsc | 80 °C | 9 | 70 |
| 7 | H | H | MeCN / H₂O (1:2) | Cul | 60 °C | 7 | 23 |

It is preferred that the reaction for obtaining the disubstituted 1,2,3-triazolylpiperidine of either general formula III or general formula IV is effected in using CH₂Cl₂ or CHCl₃ or MeCN as solvent. Of course, it is possible to use any other solvent, like tert-butanol/water, dimethylformamide, acetonitrile, etc.

### Example 2

### Synthesis of compounds of general formula (IV) in the presence of Ru catalyst

Further, the 4-ethynyl piperidine derivatives could be converted into 1,5-disubstituted 1,2,3-triazoles. Several procedures to achieve this have been described in the literature, e.g. by Krasiński et al., Org Lett 2004, 6, (8), 1237-1240. In this protocol the addition of bromomagnesium acetylide to organic azides results in the formation of 1,5-disubstituted 1,2,3-triazoles after aqueous work-up. However, when submitting the alkyne 4 to these conditions, no reaction occurred and only starting material was recovered (Table 3, entry 1).

Using the method described by Zhang et al., J Am Chem Soc 2005, 127, (46), 15998-15999, where a ruthenium catalyst is used to facilitate [1 +3] dipolar cycloaddition of acetylenes with organic azides the desired 1,5-disubstituted 1,2,3-triazoles were obtained (Table 3, entry 2 and 3). Under these conditions, both the silyl protected alcohol 4 and the unprotected alcohol 1 gave the desired products in high yield (Table 3, entry 2 and 3).

**Table 3: Synthesis of 1,5-disubstituted 1,2,3-triazoles**

| Entry | R | Solvent | Catalyst | Reaction temperature | Reaction time | Product | Isolated yield (%) |
|---|---|---|---|---|---|---|---|
| 1 | TBDMS | THF | EtMgBr | 50 °C | 36h | 11 | - |
| 2 | TBDMS | THF | Cp*RuCl(PPh₃)₂ | 80 °C | 5h | 11 | 75 |
| 3 | TBDMS | THF | Cp*RuCl(PPh₃)₂ | 80 °C | 3h | 12 | 77 |

### General procedures

NMR spectra were recorded using a Bruker Spectrospin AC with CDCl₃ as a solvent. The chemical shifts are reported in parts per million relative to TMS and the couplings constants are given in Hz. ¹³C peaks marked with * were identified from cross peaks in HSQC spectra.

Flash column chromatography was preformed using Silica gel for flash chromatography from J.T. Baker ("Baker Analyzed", 0.063 - 0.200 mm). The eluents were composed of hexanes (mixture of isomers) and ethyl acetate.

TLC analysis of the reaction mixtures was done using Macherey-Nagel aluminium plates coated with silica gel 60 with fluorescent indicator UV₂₅₄ with visualization using ultra violet light or a solution of 2% ninhydrine in ethanol containing 10 drops of conc. sulphuric acid pr. 100 mL solution.

Accurate mass determinations were performed using a Thermo Scientific LTQ Orbitrap MS.

### Example 3

### tert-Butyl trans-3-hydroxy-4-((trimethylsilyl)ethynyl)-piperidine-1-carboxylate (3).

TMS-acetylene (345 µL, 2.44 mmol) was dissolved in dry Et₂O (15 mL) and was cooled at 0°C under argon. Solutions of n-BuLi (1.6 M in hexane, 1.53 mL, 2.44 mmol) and Me₃Al (1.12 mL, 2.0 M, 2.24 mmol) were added and the reaction mixture was allowed to warm up to rt. Epoxide 2 (0.405 g, 2.04 mmol) in dry Et₂O (2 mL) was added and the reaction mixture was cooled at -78°C before BF₃ OEt₂ (0.52 mL, 4.08 mmol) was added and the reaction was stirred for 2 h at -78°C. Dry CH₃OH (3 mL) was added and after stirring for 15 min the reaction mixture was quenched with a saturated aqueous solution of NH₄Cl (10 mL). The resulting mixture was allowed to warm up to rt over a period of 30 min. H₂O was added and the mixture was extracted with Et₂O (2 x 10 mL). The organic layers were combined, dried over Na₂SO₄ and the solvent was evaporated under reduced pressure. The crude product was purified by flash column chromatography using hexane/ethyl acetate (4:1) gave the title compound as a colorless solid (0.57 g, 80%), m.p. 67.4-69.0 R_{f} (hexanes/ethyl acetate 3:1): 0.22.

¹H NMR (400 MHz, CDCl₃): δ 3.97 (m, 1 H), 3.77 (m, 1 H), 3.60 (m, 1 H), 2.99 (m, 2 H), 2.48 (m, 1 H), 2.33 (m, 1 H), 1.95 (m, 1 H), 1.56 (m, 1 H), 1.46 (s, 9 H), 0.16 (s, 9 H).

¹³C NMR (100 MHz, CDCl₃): δ 155.3, 106.4, 88.3, 80.3, 69.7, 49*, 42*, 37*, 28.7, 0.4.

HR-MS (ESI): Calcd for C₁₅H₂₇NO₃SiNa [M + Na]⁺: 320.1658, found 320.1649.

### Crystal structure of (3)

C₁₅H₂₇NO₃Si, MW = 297.47, triclinic, a = 5.7413(15), b = 11.872(3), c = 14.437(5) Å, α = 112.127(15), α = 97.874(14), γ = 98.495(10), V = 881.4(4) Å³, and d_{calc} = 1.121 g cm⁻³, *T* = 123(2) K, space group *P-1*; Z = 2. Data collected, 15714, Rᵢₙₜ 0.0185. The structure was solved by direct methods, and leastsquares refinement of the model based on 5471 independent data (4982 reflections with I > 2.0σ(I)) converged to a final R1 = 0.0321 and wR2 = 0.0885, respectively. S=1.067. Selected geometric parameters (A, °); N1-C1 1.4562(10), N1-C5 1.4646(10), N1-C11 1.3505(10), C1-C2 1.5242(11), C2-02 1.4201(10), C2-C3 1.5432(11), C3-C4 1.5370(12), C3-C6 1.4706(12), C4-C5 1.5247(12), C6-C7 1.2068(13), C7-Si1 1.8339(11), C11-O2 1.2254(10), C11-O3 1.3441(10), 03-C12 1.4675(10), C11-N1-C1 118.77(7), C11-N1-C5 123.83(7), C1-N1-C5 114.35(6), N1-C1-C2 110.96(6), O1-C2-C1 111.74(6), 01-C2-C3 107.16(6), C1-C2-C3 111.00(6), C11-O3-C12 120.39(7), C6-C3-C4 112.00(7), C6-C3-C2 109.45(7), C4-C3-C2 109.88(6), C5-C4-C3 111.30(6), N1-C5-C4 109.66(6), C7-C6-C3 176.62(9), C6-C7-Si1 172.82(8), O2-C11-O3 123.90(7), 02-C11-N1 124.53(7), O3-C11-N1 111.54(7).

### Example 4

### tert-Butyl trans-4-ethynyl-3-hydroxypiperidine-1-carboxylate (1).

Compound 3 (0.58 g, 1.95 mmol) was dissolved in TBAF (1.0 M solution in THF, 5.85 mL, 5.85 mmol). After stirring for 15 min, ethyl acetate (50 mL) was added and the reaction mixture was extracted with a saturated aqueous solution of NaHCO₃ (2 x 50 mL). The basic layers were combined and extracted with ethyl acetate (2 x 50 mL). Finally the organic layers were combined, washed with a saturated aqueous solution of NaCl, dried over MgSO₄, filtered and evaporated to dryness. Purification by flash chromatography using hexane/ethyl acetate (3:1) gave the title compound as a colorless oil (0.42 g, 95%). R_{f} (hexane/ethyl acetate 3:1): 0.16.

¹H NMR (400 MHz, CDCl₃): δ 3.95 (dd, *J* = 3.1, 1 H), 3.73 (m, 1 H), 3.62 (m, 1 H), 2.99 (m, 2 H), 2.89 (m, 1 H), 2.47 (m, 1 H), 2.19 (d, *J* = 2.1, 1 H), 1.97 (m, 1 H), 1.57 (m, 1 H), 1.45 (s, 9 H).

¹³C NMR (100 MHz, CDCl₃): δ 155.3, 84.5, 80.2, 71.5, 69.6, 48.5, 42.4, 35.7, 28.8, 28.7.

HR-MS (ESI): Calcd for C₁₂H₁₉NO₃Na [M + Na]⁺: 248.1263, found 248.1250.

### Example 5

### tert-Butyl trans-3-(tert-butyldimethylsilyloxy)-4-ethynylpiperidine-1-carboxylate (4).

Alkyne 1 (0.42 g, 1.86 mmol) was dissolved in anhydrous DMF (8 mL) and to this solution imidazole (0.152 g, 2.23 mmol) and TBDMS-Cl (0.336 g, 2.23 mmol) were added. The mixture was stirred overnight (20 hrs) at rt under argon. H₂O (10 mL) was added and the reaction mixture was extracted with ethyl acetate (2 x 10 mL). The organic layers were combined, dried over Na₂SO₄, filtered and the solvent was removed under vacuum. The resulting crude product was purified by flash column chromatography using hexane/ethyl acetate (9:1) to give colorless oil (0.598 g, 95%). R_{f} (hexane/ethyl acetate 9:1): 0.44.

¹H NMR (100 MHz, CDCl₃): δ 3.81 (m, 1 H), 3.67 (m, 1 H), 3.58 (m, 1 H), 3.12 (m, 1 H), 2.98 (m, 1 H), 2.43 (m, 1 H), 2.11 (d, *J* = 2.2, 1 H), 1.98 (m, 1 H), 1.55 (m, 1 H), 1.45 (s, 9 H), 0.90 (s, 9 H), 0.12 (s, 6 H).

¹³C NMR (100 MHz, CDCl₃): δ 155.0, 85.4, 79.9, 71.0, 70.1, 49*, 42*, 36.0, 29*, 28.8, 26.1, 18.4, -4.4.

HR-MS (ESI): Calcd for C₁₈H₃₃NO₃SiNa [M + Na]⁺: 362.2127; found 362.2148.

### Example 6

### tert-Butyl trans-3-(tert-butyldimethylsilyloxy)-4-(1-phenethyl-1H-1,2,3-triazol-4-yl)piperidine-1-carboxylate (6).

To a solution of alkyne 4 (93 mg, 0.27 mmol) and azide 5 (40 mg, 0.27 mmol) in a mixture of H₂O and CH₂Cl₂ (1:1, 6 mL) were added CuSO₄·5 H₂O (3.4 mg, 0.014 mmol) and sodium ascorbate (8.14 mg, 0.041 mmol). The reaction mixture was stirred at rt for 24 hrs. The mixture was filtered and the precipitate was washed with H₂O. The filtrate was extracted with EtOAc (3 x 10 mL) and the organic layers were combined with the precipitate. These combined layers were washed with H₂O (10 mL) and a saturated solution of NaCl in H₂O (10 mL), dried over MgSO₄, filtered, and the solvent was removed under reduced pressure. The crude product was purified by flash chromatography using hexane/ethyl acetate (1:1) to give the title compound as a colorless solid (0.113 g, 84%), m.p. 76.9-81.1 R_{f} (hexane/ethyl acetate 2:1): 0.32.

¹H NMR (400 MHz, CDCl₃): δ 7.26 (m, 3 H), 7.15 (d, *J* = 7.1, 2 H), 7.11 (s, 1 H), 4.54 (m, 2 H), 4.16 (m, 1 H), 4.06 (m, 1 H), 3.70 (m, 1 H), 3.19 (t, *J* = 7.5, 2 H), 2.74 (m, 1 H), 2.69 (m, 2 H), 1.96 (m, 1 H), 1.86 (m, 1 H), 1.47 (s, 9 H), 0.76 (s, 9 H), -0.04 (s, 3 H), -0.31 (s, 3 H).

¹³C NMR (100 MHz, CDCl₃): δ 154.9, 148.9, 137.5, 129.2, 129.0, 127.5, 122.1, 80.0, 71.5, 51.6, 51*, 44*, 42.7, 37.2, 30.4, 28.8, 26.0, 18.2, -4.4, -4.9.

HR-MS (DART): Calcd for C₂₆H₄₃N₄O₃Si [M + H]⁺: 487.3104; found 487.3115.

### Example 7

### tert-Butyl trans-3-hydroxy-4-(1-phenethyl-1H-1,2,3-triazol-4-yl)piperidine-1-carboxylate (7)

Alkyne 1 (80 mg, 0.36 mmol) was dissolved in acetonitrile (2 mL) and the mixture was heated at 60°C. Azide 5 (58 mg, 0.39 mmol) and Cul (7 mg, 0.036 mmol) were added and the reaction mixture was stirred for 40 hrs at 60°C. The reaction mixture was cooled down and the solvent was evaporated. The crude product was purified by flash chromatography to yield a colorless solid (0.122 g, 90%).

### Example 8

### tert-Butyl trans-3-hydroxy-4-(1-phenethyl-1H-1,2,3-triazol-4-yl)piperidine-1-carboxylate (7)

Compound 6 (85 mg, 0.17 mmol) was dissolved in TBAF (1.0 M solution in THF, 520 pL, 0.52 mmol) and the reaction was stirred under argon at rt. After 1 hr EtOAc (20 mL) was added and the mixture was washed with an aqueous saturated solution of NaHCO₃ (3 x 15 mL). The aqueous layers were combined and extracted with EtOAc (2 x 30 mL). The organic layers where combined, dried over MgSO₄, filtered, and the solvent was removed. Purification by flash column chromatography using hexane/ethyl acetate (1:4) gave the title compound as a colorless solid (61 mg, 96%), m.p. 135.1-136.8. R_{f} (hexane/ethyl acetate 1:4): 0.22.

¹H NMR (400 MHz, CDCl₃): δ 7.28 (m, 3 H), 7.08 (m, 2 H), 7.04 (s, 1 H), 4.58 (t, *J* = 7.2, 2 H), 4.35 (m, 1 H), 4.18 (m, 1 H), 3.60 (m, 1 H), 3.20 (t, *J* = 7.4, 2 H), 2.71 (m, 3 H), 1.91 (m, 1 H), 1.58 (m, 1 H), 1.46 (s, 9 H).

¹³C NMR (100 MHz, CDCl₃): δ 155.0, 150*, 137.3, 129.2, 129.2, 127.6, 121.3, 80.3, 70.8, 52.1, 49*, 42*, 41.3, 37.1, 28.8.

HR-MS (ESI): calcd for C₂₀H₃₀N₄O₃ [M + H]⁺: 373.2240; found 373.2261.

### Example 9

### tert-Butyl trans-3-(tert-butyldimethylsilyloxy)-4-(1-(4-methoxyphenethyl)-1H-1,2,3-triazol-4-yl)piperidine-1-carboxylate (8)

1-(Bromomethyl)-4-methoxybenzene (18 mg, 0.13 mmol) was dissolved in a mixture of CHCl₃ (0.27 mL) and a saturated aqueous solution of NaHCO₃ (0.55 mL). NaN₃ (13 mg, 0.20 mmol), Bu₄NHSO₄ (43 mg, 0.13 mmol), alkyne 4 (44 mg, 0.13 mmol), CuSO₄·5 H₂O (1.0 mg, 0.004 mmol), sodium ascorbate (3 mg, 0.013 mmol) and ethanol (0.12 mL) were added in this order and the mixture was heated to 60 °C in an oil bath. The reaction mixture was stirred for 21 hrs at this temperature and after completion cooled to rt. The reaction mixture was extracted with CHCl₃ (2 x 5 mL). The organic layers were combined, washed with H₂O (2x 5 mL), dried (MgSO₄) and the solvent was removed. Purification with flash chromatography gave the title compound as a colorless oil (47 mg, 70%). R_{f} (hexane/ethyl acetate 1:1): 0.28.

¹H NMR (400 MHz, CDCl₃): δ 7.13 (s, 1 H), 7.08 (d, *J* = 8.2, 2 H), 6.84 (d, *J* = 8.5, 2 H), 4.50 (m, 2 H), 4.13 (m, 1 H), 4.11 (m, 2 H), 3.79 (s, 1 H), 3.71 (m, 1 H), 3.12 (t, *J*= 7.8, 2 H), 2.70 (m, 3 H), 1.99 (m, 1 H), 1.85 (m, 1 H), 1.47 (s, 9 H), 0.75 (s, 9 H), -0.05 (s, 3 H), -0.33 (s, 3 H).

¹³C NMR (100 MHz, CDCl₃): δ 159.0, 148.8, 130.0, 129.4, 122.2, 114.6, 80.0, 72*, 55.6, 51.9, 51*, 43*, 42.6, 36.4, 30.0, 28.8, 26.0, 18.2, 1.4, -5.0.

HR-MS (ESI): Calcd for C₂₇H₄₄N₄O₄SiNa [M + Na]⁺: 539.3030; found 539.3013.

### Example 10

### tert-Butyl trans-3-hydroxy-4-(1-(4-methoxyphenethyl)-1H-1,2,3-triazol-4-yl)piperidine-1-carboxylate (9)

The title compound was prepared from compound 8 as described for the preparation of compound 7 to give a colorless solid (quantitative).

### Example 11

### tert-Butyl trans-3-hydroxy-4-(1-(4-methoxyphenethyl)-1H-1,2,3-triazol-4-yl)piperidine-1-carboxylate (9)

The title compound was prepared from compound 1 as described for the preparation of compound 8. The crude product was purified by flash column chromatography using hexane/ethyl acetate (1:8) to give the title compound as colorless solid (0.192 g, 70%), m.p. 107.2-109.4 R_{f} (hexane/ethyl acetate 1:8): 0.17.

¹H NMR (400 MHz, CDCl₃): δ 7.10 (s, 1 H), 6.99 (d, *J* = 8.5, 2 H), 6.82 (d, *J* = 8.5, 2 H), 4.53 (t, *J* = 7.2, 2 H), 4.35 (m, 1 H), 4.16 (m, 1 H), 3.78 (s, 3 H), 3.61 (m, 1 H), 3.14 (t, *J* = 7.2, 2 H), 2.70 (m, 3 H), 1.93 (m, 1 H), 1.59 (m, 1 H), 1.46 (s, 9 H).

¹³C NMR (100 MHz, CDCl₃): δ 159.3, 155.1, 149.6, 130.0, 129.4, 121.1, 114.7, 80.2, 70.8, 55.7, 52.3, 49.8, 43*, 41.6, 36.3, 29.4, 28.8.

HR-MS (ESI): calcd for C₂₁H₃₀N₄O₄Na [M + Na]⁺: 425.2165; found 425.2179.

### Example 12

### tert-Butyl trans-3-(tert-butyldimethylsilyloxy)-4-(1-phenethyl-1H-1,2,3-triazol-5-yl)piperidine-1-carboxylate (11)

Alkyne 1 (75 mg, 0.22 mmol) was dissolved in dry THF (3 mL) and heated at 80°C in an oil bath. To this mixture Cp*Rucl(PPh₃)₂ (1.8 mg, 0.002 mmol) and azide 5 (36 mg, 0.24 mmol) were added. The reaction mixture was stirred at 80°C for 3 hours after witch the solvent was removed using a rotary evaporator. The crude reaction mixture was purified by flash column chromatography using hexane/ethyl acetate (7:3) to give the title compound as a yellow oil (80 mg, 75%), R_{f} (hexane/ethyl acetate 7:3): 0.23.

¹H NMR (400 MHz, CDCl₃): δ 7.36 (s, 1 H), 7.24 (m, 3 H), 6.97 (m, 2 H), 4.75 (m, 1 H), 4.48 (m, 1 H), 4.13 (m, 1 H), 3.99 (m, 1 H), 3.18 (m, 3 H), 2.39 (m, 1 H), 2.28 (m, 1 H), 2.00 (m, 1 H), 1.45 (m, 10 H), 0.99 (m, 1 H), 0.74 (s, 9 H), -0.12 (s, 3 H), -0.42 (s, 3 H).

¹³C NMR (100 MHz, CDCl₃): δ 154.5, 139.9, 138.1, 130.6, 129.4, 129.1, 127.3, 80.4, 73.7, 51*, 49.8, 43*, 40.8, 37.6, 30.1, 28.7, 25.9, 18.0, -5.2.

HR-MS (ESI): calcd for C₂₆H₄₂N₄O₃SiNa [M + Na]: 509.2924; found 509.2907.

### Example 13

### tert-Butyl trans-3-hydroxy-4-(1-phenethyl-1H-1,2,3-triazol-5-yl)piperidine-1-carboxylate (12)

The deprotection of protected alcohol 11 to obtain the title compound was carried out as described for compound 7. The crude product was purified by flash column chromatography using hexane/ethyl acetate (1:9) giving the product as a colorless solid (192 mg, 75%).

### Example 14

### tert-Butyl trans-3-hydroxy-4-(1-phenethyl-1H-1,2,3-triazol-5-yl)piperidine-1-carboxylate (12)

Triazole 12 was prepared from compound 4 as described for 11. The crude product was purified by flash column chromatography using hexane/ethyl acetate (1:9) to give the title compound as a colourless solid (158 mg, 77%), m.p. 45.3-47.8. R_{f} (hexane/ethyl acetate 1:9): 0.26.

¹H NMR (400 MHz, CDCl₃): δ 7.24 (m, 3 H), 7.16 (s, 1 H), 7.01 (d, *J* = 6.5, 2 H), 4.60 (m, 2 H), 4.25 (m, 1 H), 4.00 (m, 1 H), 3.40 (m, 1 H), 3.18 (m, 2 H), 2.47 (m, 2 H), 2.13 (m, 1 H), 1.45 (m, 10 H), 1.22 (m, 1H).

¹³C NMR (100 MHz, CDCl₃): δ 154.8, 139.7, 138.2, 130.5, 129.3, 129.1, 127.3, 80.5, 71.6, 50.5, 49.8, 43.6, 40.6, 37.3, 30.3, 28.8.

HR-MS (ESI): calcd for C₂₀H₂₈N₄O₃Na [M + Na]⁺: 395.2059; found 395.2052.

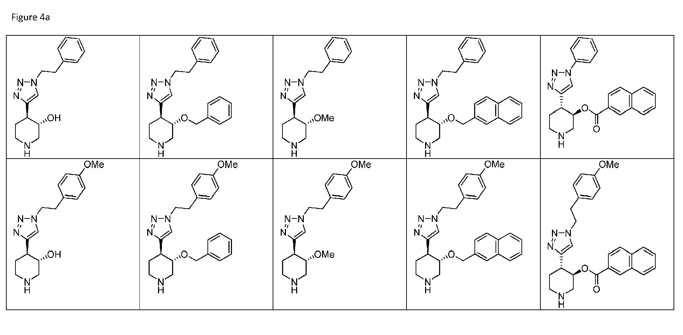

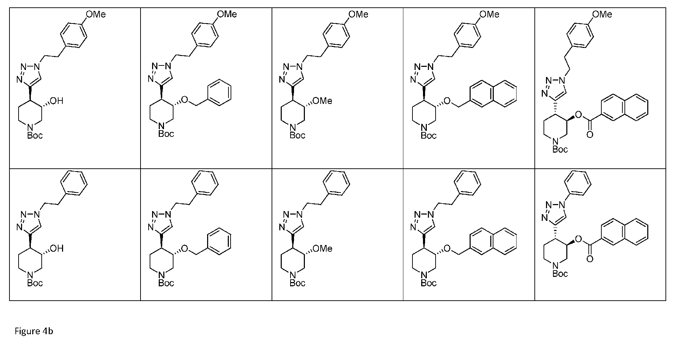

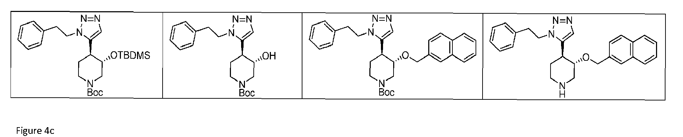

In figure 4 preferred embodiments of the compounds of general formula III and IV are shown.

## Claims

1. A compound of the general formula I 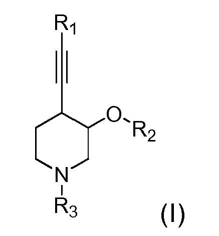 wherein R₁ is hydrogen or a protecting group,
R₂ and R₃ are independently from each other hydrogen or a protection group, or an C₁-C₁₈ alkyl group, a C₂-C₁₈ alkenyl group, a C₃-C₁₈ aryl group, a C₃-C₁₈ heteroalkyl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents, preferably, R₁ and R₂ are not hydrogen at the same time, with the proviso that R₁ and R₂ are not hydrogen at the same time when the substituents at position 3 and position 4 of the piperidine ring are in cis-form.

2. The compound according to claim 1 wherein R₁ is hydrogen and R₂ and R₃ are different protection groups.

3. The compounds according to claim 1 or 2 wherein the protection groups of R₁, R₂ and/or R₃ are selected from the group of: R₁ is a hydrogen group, R₂ is a silyl protecting group and R₃ is a carbamate protecting group, preferably a tert-butoxycarbonyl residue, or a sulfonamide, preferably a tosyl residue.

4. The compounds according to any one of the preceding claims wherein the substituents at position 3 and position 4 of the piperidine ring are in trans position.

5. Method for the synthesis of a compound according to general formula I according to any one of claims 1 to 3 comprising the step of
a) reacting an epoxide according to general formula II 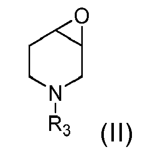 wherein R₃ is a protection group with a protected acetylide derivative, which is prepared by deprotonating a protected acetylene derivative using an alkyllithium compound, a Grignard reagent, a lithium dialkylamide or hexamethyldisilazide, preferably n-BuLi, in the presence of a trialkylaluminium reagent, like AlMe₃, and
b) cooling the reaction mixture at a temperature below room temperature, preferably at -78 °C ;
c) adding a Lewis acid, preferably, BF₃ x OEt₂, and
d) reacting the same for obtaining a compound of general formula I wherein R₂ is hydrogen.

6. The method according to claim 5 further reacting the compound obtained in step d) with a protecting agent to protect the compound of general formula I at substituent R₂ for obtaining a compound of general formula I with R₁ being hydrogen and R₂ is a protection group.

7. The method according to claim 5 or 6 wherein the reaction with the trialkylaluminium is effected at a temperature of from 0°C to room temperature and/or the reaction with the Lewis acid, in particular, BF₃ x OEt₂, is conducted at a temperature of below -50°C.

8. The method according to any one of claims 5 to 7 **characterized in** conducting the reaction of step c under Lewis acidic conditions resulting in a compound of general formula I wherein the substituent at position 3 and position 4 of the piperidine ring are in trans position.

9. An optionally substituted triazolylpiperidine compound of general formula III or IV 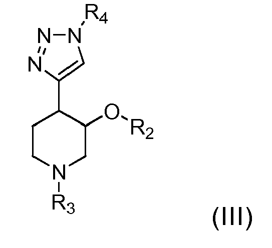 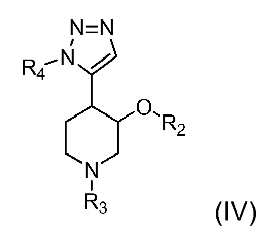 wherein R₄, R₂ and R₃ are independently from each other hydrogen or a protection group, or an C₁-C₁₈ alkyl group, a C₂-C₁₈ alkenyl group, a C₃-C₁₈ aryl group, a C₃-C₁₈ heteroalkyl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents,
R₂ and R₃ are defined as above.

10. The compound according to claim 9 wherein R₂ is selected from the group of hydrogen, C₁ to C₈ alkyl, a benzyl group,a naphtyl group, or a carbonylbenzyl group and R₃ is hydrogen.

11. The compound of general formula III or IV according to claim 9 or 10 wherein R₄ is a substituent selected from an aralkyl group optionally being substituted with an alkoxy group, like a benzyl group, optionally substituted with a methoxygroup at position 4 of the benzyl ring.

12. The compound of general formula III or IV according to any one of claims 9 to 11 wherein R₂ is a group selected from a benzyl group or a napthenmethyl group.

13. A method for obtaining an optionally substituted triazolylpiperidine according to any one of claims 9 to 12 of general formula III or IV comprising the step of reacting a compound of general formula I wherein R₁ and R₂ are as defined in claim 1 or both R₁ and R₂ are hydrogen, preferably R₁ is hydrogen, with an organic azide; and obtaining a compound of general formula III or IV.

14. The method according to claim 13 wherein the reaction of the compound of general formula I is effected in the presence of a Cu(I) catalyst or a Ru catalyst.

15. The method according to claim 13 for obtaining an 1,4-disubstituted 1,2,3-triazolylpiperidine of general formula III wherein the reaction is effected in the presence of a Cu(I) catalyst.

16. Method according to claim 13 wherein the catalyst is Ru or obtaining a 1,5-disubstituted 1,2,3-triazolylpiperidine.
